# EUROPEAN PATENT SPECIFICATION

(11) **EP 3 031 444 B1**
(45) Date of publication and mention of the grant of the patent: **10.10.2018**
(21) Application number: 16152752.8
(22) Date of filing: 24.07.2012
(51) Int. Cl.: A61K 8/06, A61K 8/25, A61K 8/64, A61K 8/81, A61Q 17/04, A61Q 19/00

(54) **OIL-IN-WATER-TYPE EMULSION COSMETIC**
ÖL-IN-WASSER-EMULSIONSKOSMETIKUM
PRODUIT COSMÉTIQUE EN ÉMULSION DE TYPE HUILE DANS EAU

(30) Priority: 02.08.2011 JP 2011169382
(43) Date of publication of application: 15.06.2016
(62) Divisional of application: 12820272.8
(73) Proprietor: SHISEIDO COMPANY, LTD., Tokyo, 104-0061 (JP)
(72) Inventor: Sato, Tomoko, Yokohama-shi, Kanagawa 224-8558 (JP); Teshigawara, Takashi, Yokohama-shi, Kanagawa 224-8558 (JP); Sugiyama, Yuki, Yokohama-shi, Kanagawa 224-8558 (JP); Kitajima, Masaki, Yokohama-shi, Kanagawa 224-8558 (JP); REGER, Martin, 95448 Bayreuth (DE); HOFFMANN, Heinz, 95448 Bayreuth (DE)
(74) Representative: Lippert Stachow Patentanwälte Rechtsanwälte

(56) References cited:
- WO-A1-2011/001633
- JP-A- 2009 234 960
- US-A1- 2011 118 382

## Description

### RELATED APPLICATIONS

This application claims the priority of Japanese Patent Application No. 2011-169382 filed on August 2, 2011.

### FIELD OF THE INVENTION

The present invention relates to an oil-in-water emulsion cosmetic, and in particular, relates to the oil-in-water emulsion cosmetic that have excellent in emulsion stability, temporal emulsion stability, and feeling in use such as the free of stickiness.

### BACKGROUND OF THE INVENTION

In many cases, emulsion cosmetics have aqueous components and oil components stably mixed by the emulsification action of added surfactants.

Meanwhile, as consumers placing greater importance on safety have increased in recent years, their demands have increased for emulsion cosmetics comprising a surfactant, which might cause skin irritation in an extremely sensitive user in rare cases or may generate stickiness, in no or a low enough content to give no such skin irritation.

The emulsions prepared by using no surfactant but by allowing powder to be adsorbed in the interface have been conventionally known as Pickering emulsions. A large number of research results have been reported up to now on the preparation of Pickering emulsions (for example, refer to Non-Patent literature 1), and a practical use thereof has been proposed in the field of cosmetics (for example, refer to Patent literature 1).

Other than the powder, some reports have also been made on the application of Pickering emulsion-like emulsions to cosmetics by using vesicles, polymer aggregates or hydrophobin derivatives which are proteins, as a material that does not dissolve in aqueous phase and oil phase (for example, refer to Patent literatures 2 to 4).

However, it has been very difficult to prepare Pickering emulsions having stability against temperature and stirring in various environments.

In addition, as for the emulsion described in Patent literature 4, some thickeners are substantially essential to provide the stability of the emulsion, but the thickeners may cause sticky or slimy feelings in use. Another problem is that the emulsion causes increase in viscosity temporally when emulsified with a hydrophobin derivative.

In this way, although there have been reported techniques for comprising an amphiphilic agent which does not dissolve in the aqueous phase and the oil phase so far for the purpose of obtaining stable emulsions, it has been difficult to obtain an oil-in-water emulsion cosmetics having sufficient stability, In addition, a new problem of the feeling in use such as sticky feeling of the cosmetic due to an amphiphilic agent has also occurred.

Patent literature 1: Japanese Unexamined Patent Publication No. 2001-518111
Patent literature 2: Japanese Unexamined Patent Publication No. 2006-239666
Patent literature 3: Japanese Patent No. 3549995
Patent literature 4: Japanese Unexamined Patent Publication No. 2009-501701

US 2011/118382 discloses an oil-in-water emulsion comprising an amphiphilic substance being a micro gel, silica having a particle size of about 100 nm, an oil component, an aqueous component and a surfactant.

JP 2009 234960 A discloses an oil-in-water emulsion comprising an amphiphilic substance being a micro gel, titanium oxide having a particle size of about 0.25 microns, an oil component, an aqueous component and a surfactant.

WO 2011/001633A1 discloses an oil-in-water emulsion comprising an amphiphilic substance being a micro gel, UV-light scattering agents in the form of particles like titanium oxide having a particle size of at most 0.1 microns, an oil component, an aqueous component and a surfactant.

Non-patent literature 1: B. Binks et. Al, Advances in Colloid and Interface Science 100-102 (2003)

### DISCLOSURE OF THE INVENTION

### PROBLEM TO BE SOLVED BY THE INVENTION

The present invention was made in view of the above-described problems, and an object of the invention is to provide an oil-in-water emulsion cosmetic having excellent in emulsion stability, temporal emulsion stability and feeling in use such as the free of stickiness although the cosmetic do not substantially comprise a surfactant.

### MEANS TO SOLVE THE PROBLEM

In order to achieve the above-described object, the present inventors have conducted a study. As a result, the present inventors have found that an oil-in-water emulsion cosmetic according to claim 1 in which a specific amphipathic substance and particles with the average particle size of less than 500 nm are comprised in combination, the particles having said particle size being selected from smectites or fluorine mica, has excellent in emulsion stability, temporal emulsion stability and feeling in use, thus leading to completion of the present invention.

That is, the oil-in-water emulsion cosmetic of the present invention is characterized by comprising the following components (a) to (d):
(a) an amphipathic substance (a3), wherein
   (a3) is a microgel that is obtained, in the dispersing phase, by radical polymerization of water-soluble ethylenically unsaturated monomers dissolved in the dispersing phase of the composition wherein an organic solvent or oil is the dispersion medium and water is the dispersing phase, and the microgel is obtained by radical polymerization of dimethylacrylamide and 2-acrylamido-2-methylpropane sulfonic acid under the conditions that the microgel forms a one-phase microemulsion or a fine W/O emulsion with a surfactant,
(b) particles with the average particle size of less than 500 nm,
(c) an oil component, and
(d) an aqueous component,
wherein the blending quantity of a surfactant is less than 0.5 mass % of the total amount of the cosmetic and wherein one or more particles with the average particle size of less than 500 nm is selected from smectites or fluorine mica.

Accordingly, in the cosmetic, component (b) is one or more kinds selected from smectites and fluorine mica.

In the cosmetic, it is preferable that the blending quantity of component (c) is 5 to 70 mass % of the total amount of the cosmetic.

In the cosmetic, it is preferable that the blending mass ratio of component (a) and component (b) is 1:20 to 2:1.

In the cosmetic, it is preferable that the blending quantity of a thickener is 0.05 mass % or less of the total amount of the cosmetic.

### EFFECT OF THE INVENTION

The oil-in-water emulsion cosmetic of the present invention comprises (a) specific amphipathic substance, (b) particles with the average particle size of less than 500 nm, (c) an oil component, and (d) an aqueous component, with the further features of claim 1, and it has an emulsion stability, temporal emulsion stability, and excellent in feeling in use.

### BEST MODE FOR CARRYING OUT THE INVENTION

The oil-in-water emulsion cosmetic of the present invention comprises (a) an amphipathic substance, namely component (a3), (b) particles with the average particle size of less than 500 nm being selected from smectites or fluorine mica, (c) an oil component, and (d) an aqueous component, and the further features of claim 1, wherein the blending quantity of a surfactant is less than 0.5 mass % of the total amount of the cosmetic.

In the following, each component is described in detail.

### ((a) Amphipathic substance

(a) An amphipathic substance incorporated in the oil-in-water emulsion cosmetic of the present invention is an amphipathic substances (a3) being a microgel that is obtained, in the dispersing phase, by radical polymerization of water-soluble ethylenically unsaturated monomers dissolved in the dispersing phase of the composition wherein an organic solvent or oil is the dispersion medium and water is the dispersing phase, and the microgel is obtained by radical polymerization of dimethylacrylamide and 2-acrylamido-2-methylpropane sulfonic acid under the conditions that the microgel forms a one-phase microemulsion or a fine W/O emulsion with a surfactant.

(a3) A microgel is a polymer microgel produced by the polymerization method generally called "revered-phase emulsion polymerization", and the details are described in Japanese Unexamined Patent Publication No. 2004-43785.

That is, its polymerization method and mechanical properties are different from those of a synthetic polymer obtained by a homogeneous polymerization system disclosed in, for example, Japanese Unexamined Patent Publication No. 2001-114641.

It is preferable to use a nonionic monomer and an ionic monomer (anionic monomer or cationic monomer) in combination as the water-soluble ethylenically unsaturated monomers that constitute the microgel. As the nonionic monomer, dialkylacrylamide represented by the following general formula (2) is preferable.

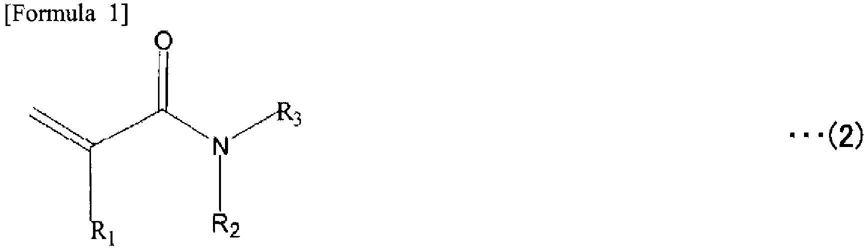

In the general formula (2), R₁ is H or a methyl group, R₂ and R₃ are a methyl group, a ethyl group, a propyl group, or a isopropyl group, respectively.

As the ionic monomer, anionic acrylamide derivative represented by the following general formula (3) or cationic acrylamide derivative represented by the following general formula (4) is preferable.

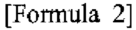

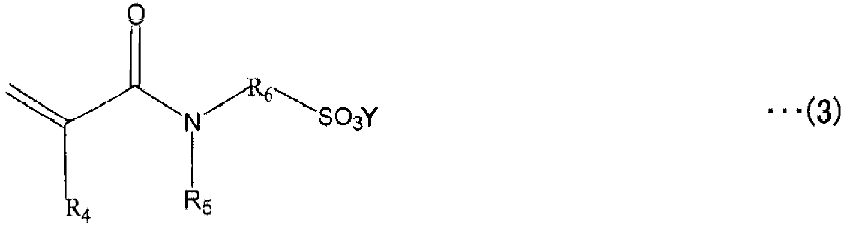

In the general formula (3), R₄ and R₅ are H or a methyl group, respectively, R₆ is a linear or branched alkyl group having 1 to 6 carbon atoms, and Y is a metal ion, NH₃, or an amine compound. Examples of metal ions include alkali metal ions such as Li, Na, and K. Examples of amine compounds include triethanol amine and triisopropanol amine.

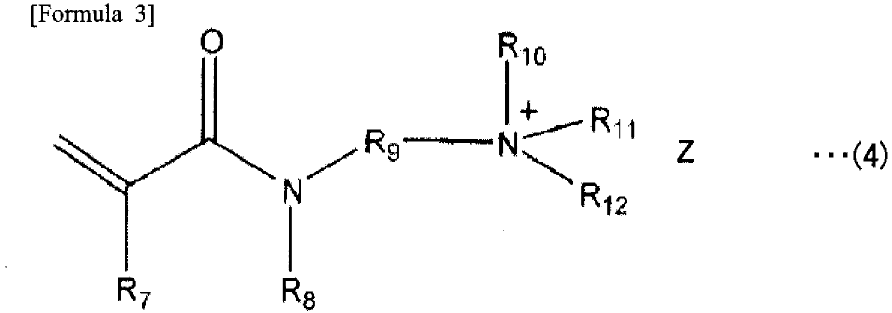

In the general formula (4), R₇ is H or a methyl group, R₈ is H or a linear or branched alkyl group having 1 to 6 carbon atoms, R₉ is a linear or branched alkyl group having 1 to 6 carbon atoms, R₁₀, R₁₁, and R₁₂ are a methyl group or an ethyl group, respectively, and Z is a negative counter ion. Examples of Z include minus counter ions such as Cl and Br.

The dialkylacrylamide is dimethylacrylamide. The ionic acrylamide derivative is 2-acrylamido-2methylpropane sulfonic acid or a salt.

The monomer composition ratio of the nonionic monomer and the ionic monomer in the polymerization system (feed ratio of the polymerization system) is selected based on the monomer composition ratio of the target microgel. The monomer composition ratio of the microgel and the feed ratio into the polymerization system are about the same.

The feed ratio of the nonionic monomer and the ionic monomer in the polymerization system (molar ratio) for copolymerization is usually in the range of nonionic monomer: ionic monomer=0.5: 9.5 to 5: 0.5, preferably 1: 9 to 9: 1, more preferably 7: 3 to 9: 1. The optimum ratio is nonionic monomer: ionic monomer=8: 2.

The aforementioned water-soluble ethylenically unsaturated monomer is then chosen at will and the microgel is polymerized. An especially preferably microgel is a dipolymer microgel copolymerized from monomers of dimethylacrylamide and 2-acrylamido-2-methylpropanesulfonic acid, used as the water-soluble ethylenically unsaturated monomer. In this case, without requiring a cross-linking monomer, a microgel that has excellent feelings in use can be obtained by self cross-linking.

A cross-linking monomer is also preferably used. Cross-linked sodium N,N-dimethylacrylamide-2-acrylamide-2-methyl propane sulfonate copolymer is also preferably used. In such case, a cross-linking monomer represented by general formula (5) is preferable, and methylenebisacrylamide is especially preferable.

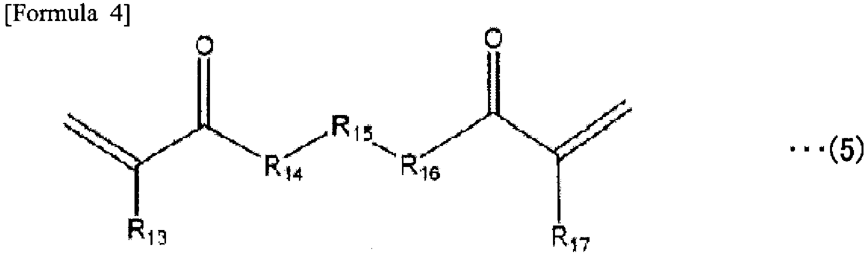

In the general formula (5), R₁₃ and R₁₇ are H or a methyl group, respectively, R₁₄ and R₁₆ are -O- or -NH-, respectively, and R₁₅ is a linear or branched alkyl group having 1 to 6 carbon atoms or -(CH₂CH₂O)ₚ- (p=4 to 100).

The blend ratio of the cross-linking monomer is preferably 0.0001 to 2.0 mol % of the total moles of 2-acrylamido-2-methylpropanesulfonic acid or its salt and dialkylacrylamide. If it is less than 0.0001 mol %, the effect of cross-linking may not be achieved. If it exceeds 2,0 mol %, the microgel cannot swell enough because the cross-link density is too high.

The weight average molecular weight of the microgel is preferably 100,000 to 5,000,000 (PEG equivalent, measured with the GPC).

The microgel has all the rheological properties listed in (1) to (3) below. This microgel is obtained by the manufacturing method according to the aforementioned polymerization method.
(1) The apparent viscosity of the microgel aqueous dispersion having 0.5 mass % of the microgel in water is 10,000 mPa·s or higher at a shear rate of 1.0 s⁻¹.
(2) The apparent viscosity of the microgel ethanol dispersion having 0.5 mass % of the microgel is 5,000 mPa·s or higher at a shear rate of 1.0 s⁻¹.
(3) The dynamic elastic modulus of the microgel aqueous or ethanol dispersion having 0.5 mass % of it satisfies the relationship G'>G" at a strain of 1% or less and a frequency range of 0.01 to 10 Hz.

The apparent viscosity of the aqueous or ethanol dispersion having the microgel is the viscosity measured with a cone/plate rheometer (MCR-300, manufactured by Paar Rhysica) at 25 °C and a shear rate of 1 s⁻¹. The dynamic elastic modulus here refers to the stored elastic modulus (G') and the loss elastic modulus (G") measured at a strain of 1% or less and a frequency range of 0.01 to 10 Hz with the aforementioned measurement apparatus at a temperature of 25 °C.

Following the polymerization, the microgel can be isolated in a powder form after easy precipitation/purification process. The microgel thus isolated in a powder form is easily dispersed in water, ethanol, or a water/ethanol mixed solvent and quickly swells. Also, for the ionic monomer to be copolymerized into the microgel, selecting a strongly acidic monomer (a monomer containing a sulfonic acid residue, for example) is preferable.

The blending quantity of (a) amphipathic substance is preferably 0.01 to 1 mass % of the total cosmetic. The blending quantity of component (a) is especially preferably 0.1 mass % or higher. If it is too small, the emulsion stability may be poor. The blending quantity of component (a) is especially preferably 0.5 mass % or less. If it is too large, the stickiness is high and the scent may not be favorable.

### ((b) Particles with the average particle size of less than 500 nm)

For (b) particles with the average particle size of less than 500 nm that is incorporated in the oil-in-water emulsion cosmetics of the present invention, the particles usually used in cosmetics can be incorporated in such a range that the stability of the emulsion is not deteriorated.

Increase in viscosity by temporal aggregation of emulsion particles and network formation of the component (a) can be inhibited by incorporating particles with the average particle size of less than 500 nm in the cosmetics of the present invention.

Such particles are selected from smectite groups (for example, synthetic hectorite, synthetic saponite, and natural bentonite) or clays (namely fluorine mica).

In the cosmetic of the present invention, it is preferable to comprise one or more particles selected from smectites or fluorine mica.

The blending quantity of (b) the particles with the average particle size of less than 500 nm is preferably 0.1 to 20 mass % of the total cosmetic. When it is too small, creaming may occur and the viscosity of the emulsion phase alone may increase temporally, allowing the cosmetics to become gel. The blending quantity of component (b) is especially preferably 5 mass % or less. When it is too large, the base material may become too hard.

In the oil-in-water emulsion cosmetics of the present invention, the blending mass ratio of the component (a) and the component (b), i.e., the blending mass of the component (a): blending mass of the component (b) is preferably 1:20 to 2:1. When the blending mass ratio of the component (a) is too small, the emulsion stability may be poor and the base material may become too hard. When the blending mass ratio of the component (b) is too small, creaming may occur and the viscosity of the emulsion phase alone may increase temporally, allowing the cosmetics to become gel.

### ((c) Oil component)

For (c) an oil component that is incorporated in the oil-in-water emulsion cosmetics of the present invention, the oil component usually used in cosmetics can be incorporated in such a range that the stability of the emulsion is not deteriorated.

Examples of liquid oils include silicone oils. Examples of silicone oils include chainlike silicones (for example, polydimethylsiloxane, methylphenylpolysiloxane, and methylhydrogenpolysiloxane) and cyclic silicones (for example, octamethylcyclotetrasiloxane, decamethylcyclopentasiloxane, and dodecamethylcyclohexasiloxane).

Examples of polar oils include ester oils (for example, octyl methoxycinnamate, cetyl octanoate, hexyl laurate, isopropyl myristate, octyl palmitate, isocetyl stearate, isopropyl isostearate, octyl isopalmitate, isodecyl isostearate, 2-ethylhexyl succinate, and diethyl sebacate).

Examples of non-polar oils include hydrocarbon oils (for example, decane, dodecane, liquid paraffin, squalane, squalene, and paraffin).

Examples of solid oils include solid fats (for example, cacao butter, coconut oil, horse fat, hydrogenated coconut oil, palm oil, beef fat, mutton suet, and hydrogenated caster oil), hydrocarbons (for example, paraffin wax (linear hydrocarbon), microcrystalline wax (branched saturated hydrocarbon), ceresin wax, Japan wax, montan wax, and Fischer-Tropsch wax), waxes (for example, beeswax, lanolin, carnauba wax, candelilla wax, rice bran wax (rice wax), spermaceti, jojoba oil, insect wax, kapok wax, bayberry wax, shellac wax, sugarcane wax, lanolin fatty acid isopropyl, hexyl laurate, reduced lanolin, hard lanolin, POE lanolin alcohol ether, POE lanolin alcohol acetate, POE cholesterol ether, lanolin fatty acid polyethylene glycol, and POE hydrogenated lanolin alcohol ether), higher fatty acids (for example, myristic acid, palmitic acid, stearic acid, and behenic acid), and higher alcohols (for example, cetyl alcohol, stearyl alcohol, behenyl alcohol, myristyl alcohol, and cetostearyl alcohols).

The blending quantity of (c) the oil component is preferably 5 to 70 mass % of the total cosmetic. The blending quantity of component (c) is especially preferably 15 mass % or higher. When it is too small, the temporal emulsion stability may be poor. The blending quantity of component (c) is especially preferably 65 mass % or less. When it is too large, the sticky feeling may be generated.

### ((d) Aqueous component)

For (d) an aqueous component that is incorporated in the oil-in-water emulsion cosmetics of the present invention, the aqueous component usually used in cosmetics can be incorporated in such a range that the stability of the emulsion is not deteriorated.

It is preferable that the cosmetic of the present invention comprises water as main component of aqueous component.

Examples of lower alcohols include ethanol, propanol, and isopropanol.

Examples of moisturizers include 1,3-butylene glycol, polyethylene glycol, propylene glycol, dipropylene glycol, hexylene glycol, glycerin, diglycerin, xylitol, maltitol, maltose, D-mannitol, and POE-POP random copolymer.

Examples of UV absorbers include benzoic acid UV absorbers (for example, p-aminobenzoic acid), anthranilic acid UV absorbers (for example, methyl anthranilate), salicylic acid UV absorbers (for example, octyl salicylate and phenyl salicylate), cinnamic acid UV absorbers (for example, isopropyl p-methoxycinnamate, octyl p-methoxycinnamate, and glyceryl mono-2-ethylhexanoate di-p-methoxycinnamate), benzophenone UV absorbers (for example, 2,4-dihydroxybenzophenone, 2-hydroxy-4-methoxybenzophenone, and 2-hydroxy-4-methoxybenzophenone-5-sulfonic acid), urocanic acid, 2-(2'-hydroxy-5'-methylphenyl) benzotriazole, and 4-tert-butyl-4'-methoxybenzoylmethane.

Examples of sequestering agents include sodium edetate, sodium metaphosphate, and phosphoric acid.

Examples of antioxidant agents include ascorbic acid, α-tocopherol, dibutylhydroxytoluene, and butylhydroxyanisol.

Examples of drugs include vitamins (for example, vitamin A oil, retinol, retinol palmitate, inositol, pyridoxine hydrochloride, benzyl nicotinate, nicotinamide, dl-α-tocopherol nicotinate, magnesium ascorbate phosphate, ascorbic acid 2-glucoside, vitamin D2 (ergocalciferol), potassium dl-α-tocopherol 2-L-ascorbate phosphate diester, dl-α-tocopherol, dl-α-tocopheryl acetate, pantothenic acid, and biotin), anti-inflammatory agents (for example, allantoin and azulene), whitening agents (for example, arbutin), astringent agents (for example, tannic acid), sulfur, lysozyme chloride, pyridoxine hydrochloride, γ-orizanol, and tranexamic acid.

The above-mentioned drugs can be used in a free state, a form of acid or basic salt if one can become salts, or a form of ester if one has a carboxylic group.

The blending quantity of (d) the aqueous component is preferably 30 to 95 mass % of the total cosmetic, and especially preferably 35 to 65 mass %.

In the oil-in-water emulsion cosmetic of the present invention, the blending quantity of thickener is preferably 0.05 mass % or less of the total cosmetic, and it is especially preferable to comprise no thickener. The incorporation of a thickener may cause sticky or slimy feelings.

Examples of the thickeners include oil-soluble thickeners corresponding to the component (c) and water-soluble thickeners corresponding to the component (d).

Examples of oil-soluble thickeners include condensation products of dibenzylidene sorbitol, tribenzylidene sorbitol, dibenzylidene xylitol or benzaldehydes with polyol having 5 hydroxyl groups or more, metallic soaps (for example, calcium stearate, calcium palimitate, litium 2-ethylhexanoate, and aluminum 12-hydroxystearate), N-acylamino acid amides (for example, lauroyl glutamate dibutylamide, lauroyl glutamate stearylamide, dicaproyl lysine laurylamine salt, dicaproyl lysine lauryl ester, and dicaproyl lysine lauryl phenylalanine laurylamide), derivatives (for example, ester and amine salt), dextrin fatty acid ester, and 12-hydroxystearic acid.

Examples of water-soluble thickeners include plant-based polymer (for example, gum arabic, tragacanth gum, galactan, guar gum, carrageenan, pectine, quince seed (Cydonia oblonga) extract, brown algae powder, and agar), microorganism-based polymer (for example, xanthan gum, dextran, and pullulan), animal-based polymer (for example, collagen, casein, albumin, and gelatine), starches (for example, carboxymethyl starch, and methylhydroxy starch), celluloses (for example, methyl cellulose, nitrocellulose, ethyl cellulose, methylhydroxypropylcellulose, hydroxyethylcellulose, cellulose sulfate, hydroxypropylcellulose, carboxymethylcellulose, crystalline cellulose, and cellulose powder), vinyl-based polymer (for example, polyvinyl alcohol, polyvinyl methyl ether, polyvinylpyrrolidone, and carboxyvinylpolymer), acrylic polymer (for example, polyacrylic acid and its salt, and polyacrylimide), glicyrrhizic acid and its salt, and alginic acid and its salt.

In the oil-in-water emulsion cosmetic of the present invention, the components usually used in cosmetics (for example, pH adjuster, preservative, antimicrobial, neutralizer, plant extract, perfume, and dye) can be incorporated in such a range that the effects of the present invention are not deteriorated,

However, in the oil-in-water emulsion cosmetic of the present invention, the blending quantity of surfactant is preferably less than 0.5 mass % of the total cosmetic, and it is especially preferable to comprise no surfactant. The incorporation of a surfactant may cause stickiness or may cause skin irritation in extremely rare cases.

Examples of surfactants include glyceryl fatty acid esters, polyglyceryl fatty acid esters, propylene glycol fatty acid esters, POE-sorbitan fatty acid esters, POE-sorbit fatty acid esters, POE-glyceryl fatty acid esters, POE-fatty acid esters, POE-alkyl ethers, POE-alkyl phenyl ethers, POE/POP-alkyl ethers, POE-castor oil derivatives, POE-hydrogenated castor oil derivatives, POE-beeswax/lanoline derivatives, alkanolamides, POE-propyleneglycol fatty acid esters, POE-alkyl amines, and POE-fatty acid amides.

The oil-in-water emulsion cosmetic of the present invention can take any of various product forms, and the examples include milky lotion, cream, foundation, sunscreen, and makeup base.

### EXAMPLES

Hereinafter, the present invention will be more concretely described by examples. However, the present invention is not limited by these examples. The blending quantity is expressed in mass % unless otherwise noted.

At first, the evaluation methods used in the present invention will be described.

### Evaluation (1): Average particle size of the emulsified particles

The average particle size of each sample (emulsion particle) immediately after the preparation was measured with a Zetasizer (Zetasizer Nano ZS, manufactured by SYSMEX CORPORATION).

### Evaluation (2): Viscosity

The viscosity of each sample (stored at 25 °C) immediately or one week after the preparation was measured with a B-type viscometer (BL-type, 12 rpm).

### Evaluation (3): Stability

The appearance of each sample immediately or one week after the preparation was observed (stored at 25 °C).
A: The sample was homogeneously liquid form.
B: Creaming (separation without coalescence of emulsion particles) was found in the sample.
C: Oil floating was observed for the sample.
D: The sample separated.

### Evaluation (4): Feeling in use

40 professional panelists conducted test by applying each sample to face and evaluated the feeling in use (absence of an oily feeling, absence of a slimy feeling during the application, and absence of stickiness after the application) based on six evaluation criteria (remarkably effective, effective, slightly effective, between effective and ineffective, slightly ineffective, and ineffective).

### (Evaluation method)

A: The ratio of the professional panelists who gave the evaluation of remarkably effective, effective or slightly effective was 50 % or higher.
B: The ratio of the professional panelists who gave the evaluation of remarkably effective, effective or slightly effective was 30 % to less than 50 %.
C: The ratio of the professional panelists who gave the evaluation of remarkably effective, effective or slightly effective was less than 30 %.

At first, Pickering emulsions using amphipathic protein are investigated (not covered by the present invention but given for reference). The present inventors prepared each oil-in-water emulsion with the blending composition shown in Table 1 in a normal method. Then, each emulsion was evaluated for the above-described evaluation criteria (1) to (3). The results are shown in Table 1.

**[Table 1]**

| Test Example | 1-1 | 1-2 | 1-3 |
|---|---|---|---|
| Wheat-derived protein (*1) | 0.5 | - | 0.5 |
| Barley-derived protein (*2) | - | 0.5 | - |
| Liquid paraffin | 35 | 35 | 65 |
| Water | balance | balance | balance |
| Average emulsion particle size (*µ*m) | 12 | 14 | 15 |
| Viscosity immediately after the preparation (mPa·s) | - | - | 6800 |
| Viscosity one week after the preparation (mPa·s) | - | - | 22000 |
| Appearance immediately after the preparation | B | B | A |
| Appearance one week after the preparation | gel (*3) | gel (*3) | gel |

| | | | |
|---|---|---|---|
| (*1): Plantasol W (manufactured by Gelita) (*2): Beer-derived (Security name: Scherdel Helle Weisse) (*3): The emulsion phase was gelation. | | | |

According to Test Example 1-1 and Test Example 1-2, with the use of wheat-derived protein, or barley-derived protein, a Pickering emulsion with a small emulsion particle size can be prepared; however, creaming is observed and gelation takes place with time.

According to Test Example 1-3, a stable emulsion can be produced immediately after preparation by increasing the blending quantity of oil; however, gelation also takes place with time.

Thus, a Pickering emulsion can be produced without the use of a surfactant by blending an amphipathic protein. However, the protein forms a network with time and thickens; therefore, it is difficult to obtain a stable emulsion.

Therefore, the components that can stabilize the emulsion wherein an amphipathic protein (not covered by the present invention) is used were investigated. The present inventors prepared each oil-in-water emulsion with the blending composition shown in Table 2 in a normal method. Then, each emulsion was evaluated for the above-described evaluation criteria (1) to (3). The results are shown in Table 2.

**[Table 2]**

| Test Example | 1-1 | 2-1 | 2-2 | 2-3 | 2-4 | 2-5 | 2-6 |
|---|---|---|---|---|---|---|---|
| Wheat-derived protein (*1) | 0.5 | 0.5 | 0.5 | - | - | - | - |
| Soybean-derived protein | - | - | - | 0.5 | - | - | - |
| Synthetic hectorite (*4) | - | 0.5 | - | 0.5 | 0.5 | - | - |
| Boehmite | - | - | 0.5 | - | - | 0.5 | 0.5 |
| POE hydrogenated castor oil | - | - | - | - | - | - | 1 |
| Liquid paraffin | 35 | 35 | 35 | 35 | 35 | 35 | 35 |
| Water | balance | balance | balance | balance | balance | balance | balance |
| Average emulsion particle size (*µ*m) | 12 | 8.9 | 6.3 | 4.8 | a few millimeters | a few millimeters | 10 |
| Viscosity immediately after the preparation (mPa·s) | - | 4200 | 6800 | 5200 | - | - | - |
| Viscosity one week after the preparation (mPa·s) | - | 4500 | 7200 | 5200 | - | - | - |
| Appearance immediately after the preparation | B | A | A | A | D | D | B |
| Appearance one week after the preparation | gel (*3) | A | A | A | D | D | B |

| | | | | | | | |
|---|---|---|---|---|---|---|---|
| (*4): Laponite XLG (manufactured by Rockwood Clay Additives GmbH) | | | | | | | |

According to Test Examples 2-1 to 2-3, an emulsion with excellent emulsion stability and a small emulsion particle size can be obtained by using an amphipathic protein in combination with particles having the average particle size of less than 500 nm, represented by synthetic hectorite and boehmite.

According to Test Examples 2-4 to 2-6, a stable and fine emulsion cannot be obtained by using only the particles with the average particle size of less than 500 nm or by using the • particles and the conventional surfactant.

Accordingly, it is necessary that the oil-in-water emulsion cosmetic of the present invention comprises (a) amphipathic substance, namely microgel (a3) according to the present invention, (b) particles with the average particle size of less than 500 nm, (c) an oil component, and (d) an aqueous component.

Subsequently, amphipathic-protein-like components, with which a stable and fine Pickering emulsion can be formed in the present invention system, were investigated. The present inventors prepared each oil-in-water emulsion with the blending composition shown in Table 3 in a normal method. Then, each emulsion was evaluated for the above-described evaluation criteria (1) to (3). The results are shown in Table 3.

**[Table 3]**

| Test Example | 3-1 | 3-2 | 3-3 | 3-4 | 3-5 | 3-6 | 3-7 |
|---|---|---|---|---|---|---|---|
| Vinylpyrrolidone/2-acrylamido-2-methylpropane sulfonic acid (salt) copolymer (*5) | 0.1 | - | - | - | 0.1 | - | - |
| Dimethylacrylamide/2-acrylamido-2-methylpropane sulfonic acid crosspolymer (*6) | - | 0.1 | - | - | - | 0.1 | - |
| Acrylic acid-methacrylic acid alkyl ester copolymer (*7) | - (*7) | - | 0.1 | - | - | - | 0.1 |
| Synthetic hectorite (*4) | 1 | 1 | 1 | 1 | - | - | - |
| Liquid paraffin | 35 | 35 | 35 | 35 | 35 | 35 | 35 |
| Water | balance | balance | balance | balance | balance | balance | balance |
| Average emulsion particle size (*µ*m) | ∼1(5) | ∼ 0.5 | ∼1(5) | ∼ 2.5(5) | ∼ 5(7.5) | ∼ 0.5(5) | ∼1(10) |
| Viscosity immediately after the preparation (mPa·s) | 5940 | 4400 | 420 | 3630 | - | - | - |
| Viscosity one week after the preparation (mPa·s) | 5450 | 4160 | 400 | 3640 | - | - | - |
| Appearance immediately after the preparation | A | A | A | C | C | C | C |
| Appearance one week after the preparation | A | A | B | C | B | B | B |

| | | | | | | | |
|---|---|---|---|---|---|---|---|
| (*5): Aristoflex AVC (manufactured by Clariant UK) (*6): SUpolymer G-1 (manufactured by TOHO Chemical Industry Co., Ltd.) (*7): PEMULEN TR-2 (manufactured by Lubrizol Advanced Materials) | | | | | | | |

According to Test Example 3-1 (not covered by the present invention but given for reference) and Test Example 3-2, when components (b) to (d) and vinylpyrrolidone/2-acrylamido-2-methylpropane sulfonic acid (salt) copolymer (not covered by the present invention) or dimethylacrylamide/2-acrylamido-2-methylpropane sulfonic acid crosspolymer, which is an amphipathic substance, are blended in the present invention, an emulsion with excellent emulsion stability and a small emulsion particle size can be obtained.

According to Test Examples 3-3 to 3-7, when only
vinylpyrrolidone/2-acrylamido-2-methylpropane sulfonic acid (salt) copolymer or only dimethylacrylamide/2-acrylamido-2-methylpropane sulfonic acid crosspolymer is used and when the conventional surfactant and component (b) are used, a stable and fine emulsion cannot be obtained.

Accordingly, it is clarified that (a3)
dimethylacrylamide/2-acrylamido-2-methylpropane sulfonic acid crosspolymer can be used according to the present invention as (a) amphipathic substance other than (a1) amphipathic protein.

Component (a3) represented by
dimethylacrylamide/2-acrylamido-2-methylpropane sulfonic acid crosspolymer needs to be a microgel that is obtained, in the dispersing phase, by radical polymerization of water-soluble ethylenically unsaturated monomers dissolved in the dispersing phase of the composition wherein an organic solvent or oil is the dispersion medium and water is the dispersing phase, and the microgel is obtained by radical polymerization of dimethylacrylamide and 2-acrylamido-2-methylpropane sulfonic acid under the conditions that the microgel forms a one-phase microemulsion or a fine W/O emulsion with a surfactant.

Subsequently, the present inventors further studied the oil-in-water emulsion cosmetic of the present invention. The present inventors prepared each oil-in-water emulsion with the blending composition shown in Table 4 in a normal method. Then, each emulsion was evaluated for the above-described evaluation criteria (2) and (4). The results are shown in Table 4.

**[Table 4]**

| Test Example | 2-1 | 4-1 |
|---|---|---|
| Wheat-derived protein (*1) | 0.5 | 0.5 |
| Synthetic hectorite (*4) | 0.5 | - |
| Carboxyvinylpolymer | - | 0.1 |
| Pottasium hydroxide | - | 0.03 |
| Liquid paraffin | 35 | 35 |
| Water | balance | balance |
| Viscosity immediately after the preparation (mPa·s) | 4200 | 5800 |
| Absence of an oily feeling | A | B |
| Absence of a slimy feeling during the application | A | C |
| Absence of stickiness after the application | A | C |

From Table 4, the sample of Test Example 2-1 of the present invention was also excellent in the feeling in use.

On the other hand, the sample of Test Example 4-1, wherein the stabilization was attempted by thickening the water phase, was poor in the feeling in use.

Accordingly, in the oil-in-water emulsion cosmetic of the present invention, the blending quantity of thickener is preferably 0.05 mass % or less, and it is more preferable to comprise no thickener.

In the following, formulation examples of oil-in-water emulsion cosmetics are listed for reference but not covered by the present invention since amphihatic proteins are used as amphilic substances instead of an amphiphatic microgel.

### Formulation Example 1: Moisturizing milky lotion

| | |
|---|---|
| Wheat-derived protein (*1) | 0.5 mass % |
| Silicon oil | 10 |
| Liquid paraffin | 20 |
| Synthetic hectorite (*4) | 1 |
| Phenoxyethanol | 0.3 |
| Dynamite glycerin | 4 |
| 1,3-butylene glycol | 5 |
| Polyethylene glycol | 3 |
| Water | balance |

### Formulation Example 2: Sunscreen milky lotion

| | |
|---|---|
| Barley-derived protein (*2) | 0.5 mass % |
| Volatile silicon oil | 10 |
| Isononyl isononate | 4 |
| Octyl methoxycinnamate | 8 |
| Butyl methoxy dibenzoylmethane | 4 |
| Hydrophobized microparticle zinc oxide | 4 |
| Microparticle titanium oxide | 2 |
| Boemite | 0.5 |
| Phenoxyethanol | 5 |
| Dipropylene glycol | 3 |
| Water | balance |

### Formulation Example 3: Whitening milky lotion

| | |
|---|---|
| Casein | 0.5 mass % |
| Silicon oil | 5 |
| Ethyl 2-ethylhexanoate | 8 |
| α-olefin oligomer | 10 |
| Synthetic saponite (Smecton SA) | 1 |
| Phenoxyethanol | 0.3 |
| Dynamite glycerin | 4 |
| POE/POP random copolymer | 4 |
| Tranexamic acid | 3 |
| Ascorbic acid | 3 |
| Water | balance |

### Formulation Example 4: Moisturing cream

| | |
|---|---|
| Wheat-derived protein (*1) | 0.5 mass % |
| Squaran | 8 |
| Jojoba oil | 5 |
| Pentaerythritol tetra 2-ethylhexanoate | 8 |
| Vaseline | 3 |
| Palm hydrogenated oil | 2 |
| Spherical silica | 3 |
| Phenoxyethanol | 0.3 |
| Dynamite glycerin | 4 |
| POE/POP random copolymer | 4 |
| POE (60) glyceryl monoisostearate | 0.1 |
| Citric acid | 0.03 |
| Sodium citrate | 0.07 |
| Water | balance |

### Formulation Example 5: Moisturing milky lotion

| | |
|---|---|
| Soybean-derived protein | 0.5 mass % |
| Isohexadecane | 10 |
| Octyl palmitate | 20 |
| Fluorine mica | 1 |
| Phenoxyethanol | 0.3 |
| Dynamite glycerin | 4 |
| Erythritol | 2 |
| Polyethyleneglycol | 3 |
| Water | balance |

## Claims

1. An oil-in-water emulsion cosmetic comprising the following components (a) to (d):
(a) an amphipathic substance (a3), wherein
(a3) is a microgel that is obtained, in the dispersing phase, by radical polymerization of water-soluble ethylenically unsaturated monomers dissolved in the dispersing phase of the composition wherein an organic solvent or oil is the dispersion medium and water is the dispersing phase, and the microgel is obtained by radical polymerization of dimethylacrylamide and 2-acrylamido-2-methylpropane sulfonic acid or its salt under the conditions that the microgel forms a one-phase microemulsion or a fine W/O emulsion with a surfactant,
(b) particles with the average particle size of less than 500 nm,
(c) an oil component, and
(d) an aqueous component,
**characterized in that** the blending quantity of a surfactant is less than 0.5 mass % of the total amount of the cosmetic and **in that** one or more particles with the average particle size of less than 500 nm is selected from smectites or fluorine mica.

2. The oil in water emulsion cosmetic according to claim 1, wherein the microgel is obtained by a feed ratio of dimethylacrylamide and 2-acrylamido-2-methylpropane sulfonic acid in the polymerization system (molar ratio) for copolymerization in the range of dimethylacrylamide : 2-acrylamido-2-methylpropane sulfonic acid = 0.5 : 9.5 to 5: 0.5.

3. The oil in water emulsion cosmetic according to claim 1 or 2, wherein the microgel is self cross-linked or cross linked by using a cross-linking monomer.

4. The oil in water emulsion cosmetic according to claim 3, wherein the blend ratio of the cross-linking monomer is preferably 0.0001 to 2.0 mol % of the total moles of 2-acrylamido-2-methylpropanesulfonic acid or its salt and dialkylacrylamide.

5. The oil in water emulsion cosmetic according to any of claims 1 to 4, wherein the weight average molecular weight of the microgel is 100,000 to 5,000,000 (PEG equivalent, measured with the GPC).

6. The oil-in-water emulsion cosmetic according to any of claims 1 to 5, wherein the blending quantity of component (c) is 5 to 70 mass % of the total amount of the cosmetic.

7. The oil-in-water emulsion cosmetic according to any of claims 1 to 6, wherein the blending mass ratio of component (a) and component (b) is 1:20 to 2:1.

8. The oil-in-water emulsion cosmetic according to any of claims 1 to 7, wherein the blending quantity of a thickener is 0.05 mass % or less of the total amount of the cosmetic.

## Patentansprüche

1. Öl-in-Wasser-Emulsionskosmetikum, umfassend die folgenden Komponenten (a) bis (d):
(a) eine amphipathische Substanz (a3), wobei
(a3) ein Mikrogel ist, das in der dispergierenden Phase erhalten wird durch radikalische Polymerisation wasserlöslicher, ethylenisch ungesättigter Monomere, die in der dispergierenden Phase der Zusammensetzung gelöst sind, wobei das Dispersionsmedium ein organisches Lösungsmittel oder Öl und die dispergierende Phase Wasser ist, und das Mikrogel durch radikalische Polymerisation von Dimethylacrylamid und 2-Acrylamido-2-methylpropansulfonsäure oder deren Salz unter Bedingungen erhalten wird, unter denen das Mikrogel eine einphasige Mikroemulsion oder eine feine Wasser-in-Öl-Emulsion mit einem oberflächenaktiven Mittel bildet,
(b) Teilchen mit einer mittleren Teilchengröße von weniger als 500 nm,
(c) eine Öl-Komponente und
(d) eine wässrige Komponente,
**dadurch gekennzeichnet, dass** der Mischungsanteil eines oberflächenaktiven Mittels weniger als 0,5 Masse-% der Gesamtmenge des Kosmetikums beträgt und dass ein oder mehrere Teilchen mit der mittleren Teilchengröße von weniger als 500 nm aus Smektiden oder Fluorglimmer ausgewählt sind.

2. Öl-in-Wasser-Emulsionskosmetikum nach Anspruch 1, wobei das Mikrogel erhalten wird durch ein Aufgabeverhältnis von Dimethylacrylamid und 2-Acrylamido-2-methylpropansulfonsäure in dem Polymerisationssystem (Molverhältnis) zur Copolymerisation im Bereich von Dimethylacrylamid:2-Acrylamido-2-methylpropansulfonsäure = 0,5:9,5 bis 5:0,5.

3. Öl-in-Wasser-Emulsionskosmetikum nach Anspruch 1 oder 2, wobei das Mikrogel selbstvernetzt oder mittels eines Vernetzungsmonomers vernetzt ist.

4. Öl-in-Wasser-Emulsionskosmetikum nach Anspruch 3, wobei das Mischverhältnis des Vernetzungsmonomers vorzugsweise 0,0001 bis 2,0 Mol-% der Gesamtmole von 2-Acrylamido-2-methylpropansulfonsäure oder deren Salz und Dialkylacrylamid ausmacht.

5. Öl-in-Wasser-Emulsionskosmetikum nach einem der Ansprüche 1 bis 4, wobei das gewichtsmittlere Molekulargewicht des Mikrogels 100.000 bis 5.000.000 (PEG-Äquivalent, gemessen mit GPC) beträgt.

6. Öl-in-Wasser-Emulsionskosmetikum nach einem der Ansprüche 1 bis 5, wobei der Mischungsanteil der Komponente (c) 5 bis 70 Masse-% der Gesamtmenge des Kosmetikums beträgt.

7. Öl-in-Wasser-Emulsionskosmetikum nach einem der Ansprüche 1 bis 6, wobei das Mischungsmasseverhältnis der Komponente (a) und der Komponente (b) 1:20 bis 2:1 ist.

8. Öl-in-Wasser-Emulsionskosmetikum nach einem der Ansprüche 1 bis 7, wobei der Mischungsanteil eines Verdickungsmittels 0.05 Masse-% oder weniger der Gesamtmenge des Kosmetikums beträgt.

## Revendications

1. Cosmétique sous forme d'émulsion huile-dans-eau, comprenant les composants (a) à (d) suivants :
(a) une substance amphipathique (a3), dans laquelle
(a3) est un microgel qui est obtenu, dans la phase de dispersion, par polymérisation radicalaire de monomères éthyléniquement insaturés solubles dans l'eau dissous dans la phase de dispersion de la composition, un solvant organique ou une huile étant le milieu de dispersion et de l'eau étant la phase de dispersion, et le microgel est obtenu par polymérisation radicalaire de diméthylacrylamide et d'acide 2-acrylamido-2-méthylpropane sulfonique ou son sel à condition que le microgel forme une microémulsion monophase ou une émulsion E/H fine avec un tensioactif,
(b) des particules ayant la dimension moyenne des particules de moins de 500 nm,
(c) un composant huileux, et
(d) un composant aqueux,
**caractérisé en ce que** la quantité de mélange d'un tensioactif est de moins de 0,5 % en masse de la quantité totale du cosmétique et **en ce qu'**une ou plusieurs particules ayant la dimension moyenne des particules de moins de 500 nm sont sélectionnées parmi des smectites ou du mica fluoré.

2. Cosmétique sous forme d'émulsion huile-dans-eau selon la revendication 1, dans lequel le microgel est obtenu par un rapport d'alimentation entre diméthylacrylamide et acide 2-acrylamido-2-méthylpropane sulfonique dans le système de polymérisation (rapport molaire) pour une copolymérisation dans la plage de diméthylacrylamide : acide 2-acrylamido-2-méthylpropane sulfonique = 0,5 : 9,5 à 5 : 0,5.

3. Cosmétique sous forme d'émulsion huile-dans-eau selon la revendication 1 ou 2, dans lequel le microgel est autoréticulé ou réticulé à l'aide d'un monomère de réticulation.

4. Cosmétique sous forme d'émulsion huile-dans-eau selon la revendication 3, dans lequel le rapport de mélange du monomère de réticulation est de préférence de 0,0001 à 2,0 % en mole des moles totales de l'acide 2-acrylamido-2-méthylpropane sulfonique ou de son sel et de dialkylacrylamide.

5. Cosmétique sous forme d'émulsion huile-dans-eau selon l'une quelconque des revendications 1 à 4, dans lequel le poids moléculaire moyen en poids du microgel est de 100 000 à 5 000 000 (équivalent PEG, mesuré à l'aide de la CPG).

6. Cosmétique sous forme d'émulsion huile-dans-eau selon l'une quelconque des revendications 1 à 5, dans lequel la quantité de mélange du composant (c) est de 5 à 70 % en masse de la quantité totale du cosmétique.

7. Cosmétique sous forme d'émulsion huile-dans-eau selon l'une quelconque des revendications 1 à 6, dans lequel le rapport en masse de mélange entre composant (a) et composant (b) est de 1 : 20 à 2 : 1.

8. Cosmétique sous forme d'émulsion huile-dans-eau selon l'une quelconque des revendications 1 à 7, dans lequel la quantité de mélange d'un épaississant est de 0.05 % en masse ou moins de la quantité totale du cosmétique.
